# EUROPEAN PATENT SPECIFICATION

(11) **EP 3 603 683 B1**
(45) Date of publication and mention of the grant of the patent: **12.06.2024**
(21) Application number: 18813779.8
(22) Date of filing: 26.04.2018
(51) Int. Cl.: A61K 33/26, A61K 33/06, A61K 9/00, A61L 24/00, A61P 7/04, A61K 35/02

(54) **IRON OXIDE/NANOKAOLIN COMPOSITE HEMOSTATIC AGENT AND PREPARATION METHOD THEREOF**
EISENOXID/NANOKAOLIN-KOMPOSIT-HÄMOSTATIKUM UND DESSEN HERSTELLUNGSMETHODE
AGENT HÉMOSTATIQUE COMPOSITE À BASE D'OXYDE DE FER/NANOKAOLIN ET SON PROCÉDÉ DE PRÉPARATION

(30) Priority: 09.06.2017 CN 201710433507
(43) Date of publication of application: 05.02.2020
(73) Proprietor: Central South University, Changsha, Hunan 410083 (CN)
(72) Inventor: YANG, Huaming, Changsha Hunan 410083 (CN); ZHANG, Yi, Changsha Hunan 410083 (CN); LONG, Mei, Changsha Hunan 410083 (CN)
(74) Representative: Petraz, Gilberto Luigi
(86) International application number: PCT/CN2018/084625
(87) International publication number: WO 2018/223790

(56) References cited:
- WO-A1-2016/133483
- CN-A- 107 213 508
- US-A1- 2013 344 131
- US-A1- 2016 325 010

## Description

### BACKGROUND

### Technical Field

The present invention relates to a hemostatic agent, and more particularly, to an iron oxide/nanokaolin composite hemostatic agent and a preparation method thereof, and belongs to the technical field of external hemostatic dressings.

### Related Art

Excessive blood loss is one of the main causes of traumatic death. The development of stable and safe hemostatic agents with fast hemostasis is of great significance for emergency rescue of trauma, especially for pre-hospital emergency such as military wounds, car accidents and natural disasters. The research and development of hemostatic materials have always been a focused issue at home and abroad. The current hemostatic materials mainly include biological hemostatic agents such as thrombin and protease, organic polymers such as chitosan and sodium alginate, inorganic substances such as silica, and mineral materials of natural silicates. Compared with other hemostatic materials, the mineral materials of natural silicate have the advantages of fast hemostasis, high stability, large yield and the like. Domestic researches on silicate hemostatic materials are mostly in a basic research stage. Among them, the main raw material of approved "XUE DUN" hemostatic powder is Ca-form zeolite. However, when zeolite adsorbs water, a large amount of adsorption heat is released, and its thermal effect may cause local tissue burns. Studies have shown that with the layered structure and surface fine pores, kaolin has good adsorption properties, can selectively absorb water in blood, effectively concentrate a substance with coagulation activity; make platelets aggregate and adhere, at the same time can stimulate blood coagulation factors and initiate an endogenous coagulation pathway to achieve coagulation purposes. In addition, kaolin has no thermal damage during hemostasis, but has an obvious defect of high hemolysis rate. The research on kaolin-containing hemostatic agents is mainly based on the combination of the kaolin as a hemostatic raw material with other hemostatic drugs such as a traditional Chinese medicine pseudo-ginseng and quercetin, or various hemostatic gauze products prepared by supporting or mixing. Red halloysite and ochre have obvious hemostatic effects in the history of traditional Chinese medicine in China, and have been applied as the main components of drugs in the pharmaceutical market. Red halloysite and ochre contain hemostatic components. However, natural minerals such as red halloysite and ochre contain high-content impurities, and thus require complicated treatment procedures, have poor biocompatibility, and are low in safety. At present, there is no report on the preparation of composite hemostatic agents by supporting iron oxide micro/nano materials on kaolin.

US 2016/325010 discloses nanoparticles, such as kaolin or iron oxides (FeO, Fe2O3, Fe3O4), adhering a biological tissue surface to a second surface to control bleeding.

### SUMMARY

### Technical Problem

In view of the above-mentioned deficiencies of the prior art, one object of the present invention is to provide a nano-scale iron oxide/nanokaolin composite hemostatic agent which has a high hemostatic rate, can promote wound healing, and has no obvious cytotoxicity, no hemolysis, and high biocompatibility.

Another object of the present invention is to provide a method for preparing the nano-scale iron oxide/nanokaolin composite hemostatic agent, as defined in claim 1, which is rich in raw materials, simple in steps, and easy to handle.

### Solution to Problem

### Technical Solution

To achieve the above technical objectives, the present invention provides a nano-scale iron oxide/nanokaolin composite hemostatic agent as in claim 1.

### Beneficial Effects of the Invention

### Beneficial Effects

Compared with the prior art, the technical solution of the present invention has the following advantages:
(1) The iron oxide/nanokaolin composite hemostatic agent of the present invention combines and enhances the hemostatic effect of both iron oxide and kaolin, and has the advantages of fast hemostasis and obvious promotion of wound healing.
(2) The iron oxide/nanokaolin composite hemostatic agent of the present invention has no obvious cytotoxicity, no hemolysis, high biocompatibility and higher safety.
(3) The raw material for the iron oxide/nanokaolin composite hemostatic agent of the present invention has wide and abundant sources, and has the advantage of low cost.
(4) The preparation method of the iron oxide/nanokaolin composite hemostatic agent of the present invention is simple in steps, easy to operate, and is advantageous for large-scale production.

### BRIEF DESCRIPTION OF THE DRAWINGS

### Description of the Drawings

[FIG. 1] is the XRD patterns of composite hemostatic agents of the present invention.
[FIG. 2] is the scanning electron micrographs of composite hemostatic agents of the present invention.

### DETAILED DESCRIPTION

### Best Mode of the Invention

To make the objectives, technical solutions and advantages of the present invention more clear, the present invention will be further described in detail below with reference to the accompanying drawings and specific examples. It should be understood that the specific examples described herein are merely illustrative of the present invention and are not intended to limit the present invention.

A nano-scale ferric oxide/nanokaolin composite of the present invention can be used for bleeding wounds, accelerates blood coagulation, and promotes wound healing.

The term "kaolin" in this specification has a chemical formula Al₂O₃·2SiO₂·2H₂O. In some forms, the kaolin includes a silica content of about 45.31%, an alumina content of about 37.21%, and a water content of about 14.1%.

The kaolin in the examples in this specification is a standard product of China Kaolin Clay Co., Ltd., and its origin is in Suzhou, Jiangsu.

### Embodiment 1

This embodiment provides preparation of nanokaolin, which was prepared by the following method:
4 g of kaolin was weighed, 10 g of potassium acetate was added, the mixture was ground for about 30 min to paste, 10% deionized water was added, and the mixture was transferred to a beaker, and allowed to stand at room temperature for three days. The mixture was washed with ethanol, filtered, and dried at 60 °C for 24 h. Potassium acetate was intercalated into kaolin, and the mixture was added to deionized water, ultrasonically treated for 30 min, washed, filtered, dried, and marked as Kaolin_{KAc}.

### Embodiment 2

This embodiment provides preparation of a polymerized hydroxy iron ion, which was prepared by the following method:
2.4 g of sodium hydroxide was weighed, and 150 mL of water was added to formulate a 0.4 mol/L sodium hydroxide solution. 10.8 g of FeCl_{3·}6H₂O was weighed, and 100 mL of deionized water was added to formulate a 0.4 mol/L FeCl₃ solution. 150 mL of a 0.4 mol/L NaOH solution was slowly added dropwise to 100 mL of 0.4 mol/L FeCl₃ solution in a 70 °C water bath under vigorous stirring. After the completion of dropwise addition, the solution was removed and slowly cooled to obtain a stable reddish-brown transparent iron polymer solution for later use, which was marked as a polymerized hydroxy iron ion solution.

### Embodiment 3 (not covered by the claim)

This embodiment provides an iron oxyhydroxide/nanokaolin composite hemostatic agent, which was prepared by the following method:
5 g of kaolin intercalated with potassium acetate was weighed and added to 250 mL of a 0.4 mol/L polymerized hydroxy iron ion solution. The pH of the reaction system was adjusted to 5 with a 5 mol/L NaOH solution. The reaction system was heated to 60 °C in a water bath, magnetically stirred for 72 h, washed, separated, and dried at 60 °C for 24 h. The product was sealed and stored in a drying vessel with allochroic silica gel at the bottom for later use, which was marked as FeOOH-Kaolin_{KAc}.

### Embodiment 4

This embodiment provides an α-ferric oxide/nanokaolin composite hemostatic agent, which was prepared by the following method:
5 g of kaolin intercalated with potassium acetate was weighed and added to 250 mL of a 0.4 mol/L polymerized hydroxy iron ion solution. The pH of the reaction system was adjusted to 5 with a 5 mol/L NaOH solution. The reaction system was heated to 60 °C in a water bath, magnetically stirred for 72 h, washed, separated, and dried at 60 °C for 24 h. The reaction system was calcined under air atmosphere at 150 °C for 1 h, at 250 °C for 1 h, and at 550 °C for 4 h. The product was sealed and stored in a drying vessel with allochroic silica gel at the bottom for later use, which was marked as α-Fe₂O₃-Kaolin_{KAc}.

### Embodiment 5

This embodiment provides an α-ferric oxide/nanokaolin composite hemostatic agent, which was prepared by the following method:
5 g of kaolin intercalated with potassium acetate was weighed and added to 250 mL of a 0.1 mol/L polymerized hydroxy iron ion solution. The pH of the reaction system was adjusted to 3 with a 5 mol/L NaOH solution. The reaction system was heated to 50 °C in a water bath, magnetically stirred for 24 h, washed, separated, and dried at 60 °C for 24 h. The reaction system was calcined under air atmosphere at 150 °C for 1 h, at 250 °C for 1 h, and at 550 °C for 4 h. The product was sealed and stored in a drying vessel with allochroic silica gel at the bottom for later use, which was marked as α-Fe₂O₃-Kaolin_{KAc}-1.

### Embodiment 6

This embodiment provides an α-ferric oxide/nanokaolin composite hemostatic agent, which was prepared by the following method:
5 g of kaolin intercalated with potassium acetate was weighed and added to 250 mL of a 0.2 mol/L polymerized hydroxy iron ion solution. The pH of the reaction system was adjusted to 5 with a 5 mol/L NaOH solution. The reaction system was heated to 60 °C in a water bath, magnetically stirred for 48 h, washed, separated, and dried at 60 °C for 24 h. The reaction system was calcined under air atmosphere at 150 °C for 1 h, at 250 °C for 1 h, and at 550 °C for 4 h. The product was sealed and stored in a drying vessel with allochroic silica gel at the bottom for later use, which was marked as α-Fe₂O₃-Kaolin_{KAc}-2.

### Embodiment 7

This embodiment provides an α-ferric oxide/nanokaolin composite hemostatic agent, which was prepared by the following method:
5 g of kaolin intercalated with potassium acetate was weighed and added to 250 mL of a 0.6 mol/L polymerized hydroxy iron ion solution. The pH of the reaction system was adjusted to 7 with a 5 mol/L NaOH solution. The reaction system was heated to 70 °C in a water bath, magnetically stirred for 72 h, washed, separated, and dried at 60 °C for 24 h. The reaction system was calcined under air atmosphere at 150 °C for 1 h, at 250 °C for 1 h, and at 550 °C for 4 h. The product was sealed and stored in a drying vessel with allochroic silica gel at the bottom for later use, which was marked as α-Fe₂O₃-Kaolin_{KAc}-6.

### Embodiment 8

This embodiment provides a γ-ferric oxide/nanokaolin composite hemostatic agent, which was prepared by the following method:
5 g of kaolin intercalated with potassium acetate was weighed and added to 250 mL of a 0.4 mol/L polymerized hydroxy iron ion solution. The pH of the reaction system was adjusted to 5 with a 5 mol/L NaOH solution. The reaction system was heated to 60 °C in a water bath, magnetically stirred for 72 h, washed, separated, and dried at 60 °C for 24 h. The reaction system was calcined under air atmosphere at 150 °C for 1 h, at 250 °C for 1 h, and at 550 °C for 4 h. The reaction system was calcined in high purity argon containing 10% hydrogen at 450 °C for 1 h and calcined in an air atmosphere at 250 °C for 1 h. The product was sealed and stored in a drying vessel with allochroic silica gel at the bottom for later use, which was marked as γ-Fe₂O₃-Kaolin_{KAc}.

### Embodiment 9

This embodiment provides a ferroferric oxide/nanokaolin composite hemostatic agent, which was prepared by the following method:
5 g of kaolin intercalated with potassium acetate was weighed and added to 250 mL of a 0.4 mol/L polymerized hydroxy iron ion solution. The pH of the reaction system was adjusted to 5 with a 5 mol/L NaOH solution. The reaction system was heated to 60 °C in a water bath, magnetically stirred for 72 h, washed, separated, and dried at 60 °C for 24 h. The reaction system was calcined under air atmosphere at 150 °C for 1 h, at 250 °C for 1 h, and at 550 °C for 4 h. The reaction system was calcined in high purity argon containing 10% hydrogen at 450 °C for 1 h. The product was sealed and stored in a drying vessel with allochroic silica gel at the bottom for later use, which was marked as Fe₃O₄-Kaolin_{KAc}.

### Embodiment 10

This embodiment provides an α-ferric oxide particle, which was prepared by the following method:
250 mL of a 0.4 mol/L polymerized hydroxy iron ion solution was added to a conical flask. The pH of the reaction system was adjusted to 5 with a 5 mol/L NaOH solution. The reaction system was heated to 60 °C in a water bath, magnetically stirred for 72 h, washed, separated, and dried at 60 °C for 24 h. The reaction system was calcined under air atmosphere at 150 °C for 1 h, at 250 °C for 1 h, and at 550 °C for 4 h. The product was sealed and stored in a drying vessel with allochroic silica gel at the bottom for later use, which was marked as α-Fe₂O₃.

### Application Embodiment

Hemostasis experiment: BALB/C mice (6 weeks old) were selected as experimental animal subjects. The rats were randomly divided into groups, each group including six rats, and the rats used for experiment were fed normally without restriction. A surgical scissor was used to make an 1 cm long wound at the tail vein. After the bleeding, a sample was applied to cover the wound sufficiently. When a medicine was applied, the bleeding wound was slightly pressed, and the bleeding time was recorded with a stopwatch. The hemostasis standard was that there was no ejection and oozing of blood on a wound surface, namely, the blood was clotted and no longer re-oozed.

Wound healing: Before a wound treatment experiment, an experimental rat was first anesthetized by intramuscular injection of 35.0 mg/kg ketamine and 5.0 mg/kg xylazine, and fixed on a rat fixation plate. The back of the rat was subjected to depilation treatment, and the effective area of depilation was about (2 cm × 2 cm), and the depilated back of the rat was washed with ethanol.

The surgical scissor and forceps were used to make a wound (1.0 cm × 1.0 cm) on the depilated and cleaned back of the rat. The blood flowing out of the wound was wiped off, composite hemostatic sample powder was immediately spread on the wound, and the wound was wrapped with bandages and gauze in sequence. The wound healing process of each composite hemostatic agent was recorded using a digital camera. The medicine (composite hemostatic agent) was changed every 2-3 days, and the healing of the wound was observed.

Histological analysis of healed wounds: After 10 days, the wound was basically healed. The tissue at the wound was cut, fixed by directly immersing in 4% paraformaldehyde, embedded, sliced, and stained with a hematoxylin-eosin reagent (H&E), Masson, and Sirius Red (SR), respectively to observe the tissue healing in each group.

Cytotoxicity of composite hemostatic agent: Fibroblast L929 cells were selected to evaluate the biocompatibility of the composite hemostatic agent. Fibroblasts were cultured using Dulbecco's Modified Eagle Medium (specifically, 10% fetal bovine serum, 2 mmol/L glutamine, 4.5 g/L glucose, and 1% antibiotic/antimycotic solution). The fibroblasts were cultured at 37 °C in a 5% CO₂ sterile environment. The medium was replaced with a fresh medium every two days until the cells reach a level of aggregation.

Evaluation of cytotoxicity by MTT assay: The composite hemostatic agent was added to a 24-well plate, and each well was inoculated with 1 mL of cell suspension at a concentration of 2×10⁴ cells/mL, and the concentration of the composite hemostatic agent was 0~2 mg/mL. A 100 µL MTT solution was injected into each well, and then the cells were cultured for 4 h. Once the culture was completed, supernatant was removed, and 200 µL of DMSO was added to give a blue reduction product, formazan. The dissolved solution was then transferred to a 96-well plate and the absorbance was measured with a microplate reader (570 nm). Three parallel experiments were performed. The group only with a culture solution was used as a blank group, and the group with the cells and the culture solution was used as a control group to correct the cell survival rate.

Hemolysis test: 80 mg of the composite hemostatic agent was mixed with 0.4 mL of a 10% red blood cell solution, and allowed to stand at room temperature for 1 h. 150 µL was taken into a 96-well plate, and the absorbance was measured with a microplate reader (540 nm). Water and PBS were used as positive and negative controls, respectively. Hemolysis rate (%)=(sample absorbance-negative control absorbance)/(positive control absorbance - negative control absorbance) × 100%.

### Experimental results:

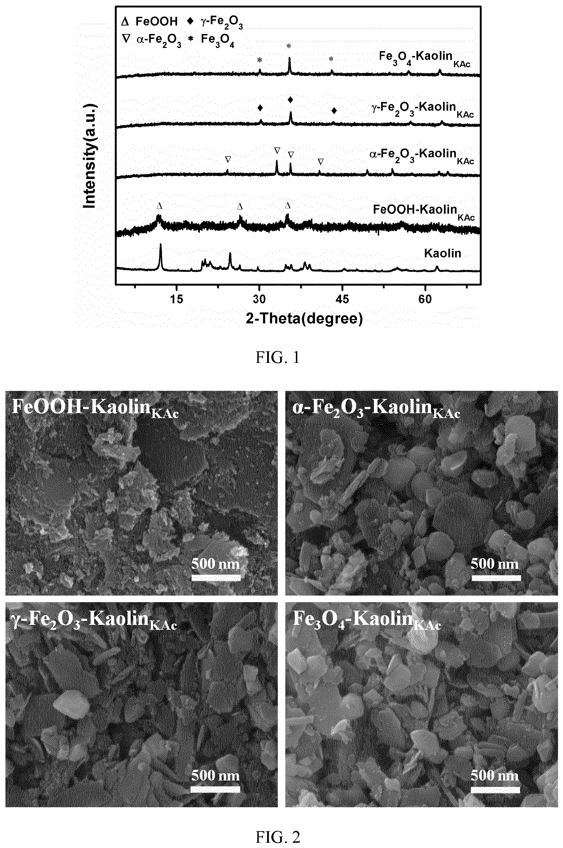

Preparation of iron oxide/nanokaolin composite hemostatic agent: upon identification by an X-ray diffraction technique, as shown in FIG. 1, the samples of iron oxyhydroxide/nanokaolin, α-ferric oxide/nanokaolin, γ-ferric oxide/nanokaolin, and ferroferric oxide/nanokaolin obtained in Embodiment 3, Embodiment 4, Embodiment 8, and Embodiment 9 have characteristic diffraction peak of iron oxyhydroxide, α-ferric oxide, γ-ferric oxide, and ferroferric oxide, respectively. As shown in FIG. 2, the kaolin retains a complete pseudo-hexagonal flake structure, and the iron oxyhydroxide in example 3 has a spindle-like morphology and a particle size of about 100 nm. The iron oxides of Embodiment 4, Embodiment 8, and Embodiment 9 are polyhedrals, and small particles are supported on the surface and sides of the kaolin flake. In addition, some of the particles form large particles having a particle size of about 300 nm during calcination.

Hemostasis experiment: The hemostatic time of the blank control group, kaolin and the samples of iron oxyhydroxide/nanokaolin, α-ferric oxide/nanokaolin, γ-ferric oxide/nanokaolin, and ferroferric oxide/nanokaolin obtained in Embodiment 3, Embodiment 4, Embodiment 8, and Embodiment 9 are 313±23 s, 227±17 s, 298±14 s, 183±16 s, 212±11 s, and 218±15 s, respectively. The samples have a better hemostatic effect than the blank control group and kaolin alone.

The hemostatic time of kaolin and the α-ferric oxide/nanokaolin composites with different α-ferric oxide mass fractions prepared in Embodiment 5, Embodiment 6, Embodiment 4, Embodiment 7, and Embodiment 10 are 227±17 s, 158±21 s, 132±13 s, 183±16 s, 216±13 s, and 193+9 s, respectively. The corresponding α-ferric oxide mass fractions are 0, 15.7%, 23.9%, 43.6%, 49.4%, and 100%, respectively.

Wound healing: The kaolin and the samples of iron oxyhydroxide/nanokaolin, α-ferric oxide/nanokaolin, γ-ferric oxide/nanokaolin, ferroferric oxide/nanokaolin and α-ferric oxide particle obtained in Embodiment 3, Embodiment 4, Embodiment 8, Embodiment 9, and Embodiment 10 accelerate wound healing, reduce inflammation, and promote tissue remodeling.

Cytotoxicity: The cytotoxicity of each composite hemostatic agent was evaluated for fibroblasts as an object. The kaolin and the samples of iron oxyhydroxide/nanokaolin, α-ferric oxide/nanokaolin, γ-ferric oxide/nanokaolin, ferroferric oxide/nanokaolin and α-ferric oxide particle hemostatic agent obtained in Embodiment 3, Embodiment 4, Embodiment 8, Embodiment 9, and Embodiment 10 have almost no toxicity to cells and have good biocompatibility.

Hemolysis: Kaolin has a high hemolysis rate of 30% and hemolysis. The hemolysis rates of the samples of iron oxyhydroxide/nanokaolin, α-ferric oxide/nanokaolin, γ-ferric oxide/nanokaolin, ferroferric oxide/nanokaolin and α-ferric oxide particle obtained in Embodiment 3, Embodiment 4, Embodiment 8, Embodiment 9, and Embodiment 10 are all less than 5%, indicating that no hemolysis is caused.

## Claims

1. An iron oxide/nanokaolin composite hemostatic agent, wherein an active ingredient of the composite hemostatic agent is made by compounding nanokaolin and nano-scale iron oxide,the iron oxide being supported on a surface of the nanokaolin, the mass percentage of the iron oxide being 15%-45%,wherein the iron oxide is α-ferric oxide, γ-ferric oxide and/or ferroferric oxide.

## Patentansprüche

1. Aus Eisenoxid/Nanokaolin zusammengesetztes Hämostatikum, worin ein Wirkstoff des zusammengesetzten Hämostatikums durch Mischung von Nanokaolin und nanoskaligem Eisenoxid erzeugt wird, wobei das Eisenoxid auf einer Oberfläche des Nanokaolins unterstützt ist, der Massenanteil des Eisenoxids 15%-45% beträgt, worin das Eisenoxid *α*-Eisen(III)-Oxid, *γ*-Eisen(III)-Oxid und/oder Ferroferricoxid ist.

## Revendications

1. Agent hémostatique composite d'oxyde de fer/nano-kaolin, dans lequel un ingrédient actif de l'agent hémostatique composite est réalisé en combinant du nano-kaolin et de l'oxyde de fer à l'échelle nanométrique, l'oxyde de fer étant supporté sur une surface du nano-kaolin, le pourcentage massique de l'oxyde de fer étant de 15% à 45%, dans lequel l'oxyde de fer est l'oxyde α-ferrique, l'oxyde γ-ferrique et/ou l'oxyde ferroferrique.
